# EUROPEAN PATENT SPECIFICATION

(11) **EP 3 975 842 B1**
(45) Date of publication and mention of the grant of the patent: **19.06.2024**
(21) Application number: 20728061.1
(22) Date of filing: 28.05.2020
(51) Int. Cl.: A61B 5/1455, A61B 5/00

(54) **APPARATUS FOR MEASURING OPTICAL OR PHYSIOLOGICAL PARAMETERS IN HUMAN TISSUE FEATURING AN OPTICAL CONTACT DETECTOR**
VORRICHTUNG ZUR MESSUNG OPTISCHER ODER PHYSIOLOGISCHER PARAMETER IN MENSCHLICHEM GEWEBE MIT EINEM OPTISCHEN KONTAKTDETEKTOR
APPAREIL DE MESURE DE PARAMÈTRES OPTIQUES OU PHYSIOLOGIQUES DANS LE TISSU HUMAIN COMPORTANT UN DÉTECTEUR DE CONTACT OPTIQUE

(30) Priority: 28.05.2019 EP 19177063
(43) Date of publication of application: 06.04.2022
(73) Proprietor: Universität Zürich, 8006 Zürich (CH)
(72) Inventor: KLEISER, Stefan, 79639 Grenzach-Wyhlen (DE); OSTOJIC, Daniel, 8046 Zürich (CH); WOLF, Martin, 8038 Zürich (CH)
(74) Representative: Mertzlufft-Paufler, Cornelius
(86) International application number: PCT/EP2020/064862
(87) International publication number: WO 2020/239920

(56) References cited:
- DE-U1- 29 924 810
- US-A1- 2009 163 787
- US-A1- 2014 155 753
- US-A1- 2019 069 781
- US-B1- 6 230 046

## Description

The background of the invention concerns an apparatus for measuring at least one optical or physiological parameter in a scattering medium, in particular in human tissue, comprising at least one light source for emitting at least one measurement wavelength Am, at least one detector for detecting said at least one measurement wavelength, and an electronic unit configured to measure a measurement light quantity resulting from an emission of said at least one measurement wavelength into said medium, wherein said measurement is based on electrical signals read-out from said at least one detector and serves the purpose of computing said at least one parameter. Such a measurement light quantity may be an intensity, a phase, or a time of flight, for example.

The background of the invention further concerns a method for evaluating an optical coupling efficiency between a contact area of an apparatus designed for measuring at least one optical or physiological parameter in a scattering medium, and a surface of said medium, wherein at least one measurement wavelength is emitted by said apparatus through said contact area into said medium and received through said contact area by said apparatus after having propagated through said medium, and wherein a corresponding measurement light quantity resulting from said emission of said measurement wavelength is measured by said apparatus.

Such apparatuses and methods are already employed in clinical use for measuring cerebral oxygenation based on near infrared spectroscopy (NIRS), in particular for vital-parameter-monitoring of infants. For this purpose, a sensor head of the apparatus, bearing a flexible measuring printed circuit board on which light sources and detectors are arranged, is bent around the head of the infant to achieve good skin contact, which is crucial for a reliable measurement that delivers true oxygenation values.

It has been observed that in clinical use, the sensor head of the apparatus sometimes detaches from the skin, due to a movement of the infant, for instance. This may be case even if the sensor head is securely fixed to the skull by adhesive tapes or the like. In consequence, an air gap may form between a contact area on the lower side of the sensor head, which is normally in skin contact during a measurement, and the skin on the skull of the infant. As a result of such a situation, the measured oxygenation will be false, as part of the light path is in air and not in the tissue, as assumed. Hence, the monitoring of the oxygenation will be incorrect. Such incorrect monitoring can pose a severe threat to the life of the infant and is therefore to be avoided.

Concerning prior art, documents US 2019/0069781 A1, US 2014/0155753 A1 and US 2010/0094106 A1 reveal, respectively, optical sensors which employ LEDs and photodiodes for measuring physiological parameters such as heart rate or oxygenation levels. These devices also feature basic technical means for measuring a contact or distance between the respective sensor and the skin. For avoiding interference between a measurement wavelength (used for measuring a physiological parameter or for visualization of veins and arteries) and a detection wavelength (used for optically evaluating a tissue contact) US 2014/0155753 A1 proposes a time-multiplexing-approach, in which the respective measurement and detection wavelength are emitted without overlap in time.

Document US 2009/0163787 A1 describes a myriad of different (electrical and/or optical) techniques for measuring a tissue contact with a respective "tissue contact sensor".

It is also known that reaching a defined distance from a target surface can be detected by optical means using a contact-less optical measuring approach, as presented in DE 299 24 810 U1.

Starting out from this background, it is an object of the present invention to provide an apparatus which alleviates the afore mentioned deficiency. In particular, the invention aims at providing an optical contact detection scheme which allows robust detection of a proper skin contact of the sensor while the sensor is measuring, in particular without using a time-multiplexing approach.

In accordance with the present invention, an apparatus is provided according to claim 1, which solves the aforementioned problem. An apparatus as introduced at the beginning is thus proposed, which, in addition, is characterized in that the apparatus features an optical contact detector for detecting a detection wavelength, which is different from said at least one measurement wavelength. This optical contact detector features an optical filter transmitting said detection wavelength and blocking said at least one measurement wavelength. Accordingly, the detection of a skin contact can be performed, independently of, in particular simultaneously to, the measurement of the measurement light quantity. A time-multiplexing approach is thus not necessary. Moreover, the detection wavelength lies outside of the NIR-range from 640 to 1350 nm, and the electronic unit of the apparatus is configured to measure a detection light quantity based on electrical signals outputted by said optical contact detector and to detect a direct skin contact of the apparatus based on the measured detection light quantity. The at least one light source, the at least one detector and the optical contact detector are arranged on a contact area of the apparatus, in particular on the lower side of a bendable sensor head of said apparatus, which is brought into contact with skin during a measurement.

In other words, said electronic unit is configured to measure a detection light quantity resulting from said detection wavelength, based on electrical signals outputted by said optical contact detector, and to detect a contact state based on the measured detection light quantity.

According to a specific embodiment, the electronic unit may be configured, in addition, to derive a variable from said detection light quantity and to compute a deviation of said variable from a target value. In such an embodiment, a high safety in using the apparatus may be achieved if the electronic unit is configured to issue an alarm signal in case said deviation is greater than a threshold value.

To sum up, the invention suggests that said apparatus introduced at the beginning features an optical contact detector designed for detecting a specific detection wavelength. This optical contact detection serves the purpose of determining a contact state of the apparatus, which may be at a far distance from, in loose contact to, or in close contact to the medium to be investigated.

As outlined at the beginning, the electronic unit may be configured to measure a measurement light quantity resulting from an emission of said at least one measurement wavelength into the medium, based on electrical signals read-out from said at least one detector. Moreover, the electronic unit may also be configured to compute said at least one optical or physiological parameter from said measurement light quantity.

For enabling NIR-measurements in deep tissue layers, it is preferable if said detector for detecting said at least one measurement wavelength is positioned at a distance of at least 12 mm, preferably at least 15 mm, from said at least one light source for emitting at least one measurement wavelength. In other words, it is preferable if said apparatus features at least one source-detector-separation (SDS) of at least 12 mm, preferably at least 15 mm, for determining the optical or physiological parameter in the scattering medium, which may be human tissue.

The detection wavelength lies thus outside of a wavelength band used by the apparatus for determining said optical or physiological parameter.

As will be explained in more detail below, these features are meaningful, because the invention suggests that the detection wavelength lies in a wavelength band that is not appropriate for measuring said optical parameter. This may be due to the fact, that the detection wavelength may show an attenuation in said medium (due to scattering and/or absorption) that is too large for a meaningful measurement of said optical parameter.

A large attenuation of the detection wavelength in the medium is, however, beneficial for obtaining a robust optical contact detection mechanism, using the optical contact detector, because a large attenuation can be more easily and more accurately detected when the device is not in full contact with the medium as compared to a small attenuation, in particular when the detection scheme is based on determining an optical absorption or a concentration of a chromophore in said medium. Using a separate detection wavelength is thus key for safeguarding a high measurement accuracy for determining the optical parameter and a high safety of the measurement by offering an efficient and robust optical contact detection mechanism.

Said electronic unit is configured to detect a close contact state (e.g., direct skin contact of the apparatus) based on the measured detection light quantity, wherein the measured detection light quantity exclusively results from the detection wavelength but not from the at least one measurement wavelength. Hence, the electronic unit detects the close contact state without employing or relying on said at least one measurement wavelength.

According to a specific embodiment, the electronic unit may be configured to measure two separate detection light quantities each resulting from a different detection wavelength. In this case, the electronic unit may determine the contact state of the apparatus with respect to the medium to be measured by measuring and/or comparing these separate detection light quantities. For example, if the detection light quantities are light intensities, the electronic unit may compute respective optical absorptions from these intensities and compare them. Such comparison can then be used by the electronic unit for performing a validity or plausibility check of the optical contact measurement. For example, in close contact, the ratios of the absorptions should be close to those expected for the specific medium / tissue investigated and for the specific detection wavelengths used.

From output signals of the optical contact detector, the electronic unit may compute the detection light quantity.

The detection light quantity measured by the electronic unit can be a detection light intensity and/or a detection light phase and/or a detection light time of flight. Such quantities are useful, because conclusions can be drawn from such quantities on an average optical path length (OPL) that photons of the detection wavelength have traveled through the medium, in particular through tissue, before reaching the optical contact detector.

For example, the longer the OPL, the stronger will be the attenuation of the detection light due to absorption and/or scattering in the tissue. Likewise, the phase shift will be larger, the longer the OPL inside the tissue. A long OPL indicates an absence of air gaps between the apparatus and the skin surface and hence a good contact, i.e., a high optical coupling efficiency between the apparatus and the tissue. Generally, a long OPL inside the tissue will result in a strong attenuation and hence low detection light intensity and/or a large phase shift.

In consequence, the detection light phase may be used alternatively or additionally to a measured detection light intensity to determine the state of contact between the apparatus and the surface of the medium to be measured. However, using a detection light intensity as the detection light quantity is generally preferable for measurements in a highly absorbing medium, because in case the absorption of the detection wavelength in the medium is very high, the signal-to-noise ratio will be so low that phase measurements are no longer feasible.

In addition, there exists a technique called time domain NIRS, which measures the time of flight (TOF) of photons and which can also be used by the apparatus according to the invention for measuring said optical parameter. For example, the apparatus may feature a light source for emitting ultra-short pulses of several measurement wavelengths, for example in the picosecond to femtosecond range, and the electronic unit may be configured to measure a corresponding time point spread function (TPSF) of this measurement light, after it has travelled through the tissue. Based on the information of the distribution of the arrival times of photons, which can be derived from the TPSF, optical parameters such as absolute absorption and scattering coefficients, and from these absolute concentrations of chromophores in the tissue, may be measured with the apparatus.

Moreover, this approach can not only be used for measuring said optical parameter but for measuring said detection light quantity. In other words, the apparatus may feature a light source for emitting ultra-short pulses of a detection wavelength, which may be said light source described above, and the electronic unit may be configured to measure a corresponding time point spread function (TPSF) of this detection light, in particular using said optical contact detector, after the detection light has travelled through the tissue.

The longer the arrival time of a photon, the higher the probability that the photon has probed deep tissue. Hence, by separating early from late photons, corresponding to superficial and deep optical paths through the tissue, respectively, it is possible to draw conclusions on the average OPL inside the tissue. Therefore, the optical contact detector and the electronic unit may be configured to measure a TOF of the detection light, i.e. a TOF of photons of the detection wavelength which reach the optical contact detector. From this TOF, conclusions can be drawn on the average OPL of photons in the tissue, because, due to scattering in the tissue, the TOF will increase the longer the photons have traveled through tissue.

In consequence, a comparatively long TOF can be indicative of an absence of air gaps between the apparatus and the skin surface and hence of a good skin contact.

As was outlined above, the electronic unit may be further configured to derive a variable from said detection light quantity; this variable may be, for example, a detection light intensity and/or a phase of the detection light and/or a TOF of the detection light or a variable derived from such quantities, for example a variable derived from the detection light intensity measured by said optical contact detector.

In the simplest case, the variable may be the detection light quantity itself, for example a detection light intensity measured by the optical contact detector.

If the detection wavelength is strongly scattered and/or absorbed in the medium (which will be the case for example for blue light which is strongly scattered), in fact no detection light at all may reach the contact detector, when the apparatus is in close contact to the medium, in particular tissue/skin. Such an absence of a detection signal from the detection wavelength, which may be still considered a "measurement", may thus be interpreted by the apparatus as a good contact state.

In yet other application cases, a weak detection signal, for example below a certain threshold value, may already be interpreted by the apparatus as representing a full contact. For this purpose, the calculation of the variable computed by the apparatus may be based on some modeling of the light propagation of the detection wavelength in the medium. Such modeling / calculation may also comprise a plausibility check of the measurement obtained with the detection wavelength. In any case, the amount of detection signal that is present at the optical contact detector will depend on the chosen detection wavelength, the placement of sources and detectors as well as the properties of the tissue optically probed with the detection wavelength. Thus, the operation mode of the contact detector will depend on the specific application case.

In addition, due to scattering in the medium and propagation of the detection wavelength in the medium, the detection wavelength may be re-emitted from the medium at other locations than the location of incidence. Thus, when the apparatus determines its contact state in close contact using the optical contact detector, the detection wavelength may reach the optical contact detector only after having traveled along some distance through the medium / the tissue and after having been re-emitted by said medium / said tissue but no longer due to direct reflection from a surface of said medium / from skin.

In other words, the apparatus may be configured to detect the detection wavelength in a non-reflective measurement mode, in which the detection wavelength reaches the detector when brought in close contact to said surface only after having traveled through said medium / tissue for some distance, for example for at least 5 mm, preferably for at least 12 mm. This can be safeguarded, for example, if the optical contact detector is positioned at a distance of at least 5 mm, preferably of at least 12 mm, from a detection light source for emitting said detection wavelength or from a border of the contact area, described previously. When using short wavelengths, the distance may be smaller, however.

For robust optical contact detection in a non-reflective measurement mode, it is also preferable if the optical contact detector and the detection light source are each mounted in a separate cavity. This approach ensures that no direct optical crosstalk can happen between the optical contact detector and the detection light source.

In yet other application cases, the variable may be an optical absorption or a concentration of a chromophore, for example.

Accordingly, the electronic unit may be configured to compute said variable from said detection light quantity.

The variable derived from the detection light quantity, i.e. from a detection light intensity and/or a detection light phase and/or a detection light TOF, may be used by the electronic unit for evaluating the contact between the apparatus at the location of the optical contact detector and a surface of said medium, for example the skin on the head of a neonate whose cerebral tissue is optically and non-invasively monitored with the apparatus. A good contact between the apparatus and the skin is tantamount to a high optical coupling efficiency, which is a necessary condition for a reliable measurement of said parameter.

The underlying mechanism is that the detection wavelength will be stronger attenuated due to absorption in the tissue, when the apparatus is in direct contact with the skin, as compared to a situation, in which there is an air gap between the apparatus and the skin. Likewise, the phase shift will be larger, the longer the light has travelled in the tissue. Additionally, as has been explained above, an average TOF will tend to be longer, the longer the OPL inside the tissue.

Depending on a comparison of said variable with a target value indicating a full skin contact, the electronic unit may then issue a control signal indicating an insufficient skin contact, i.e., insufficient for accurately determining the true value of said parameter measured with the apparatus.

As a major benefit of this approach, the apparatus may thus warn a user in case the skin contact of the apparatus is insufficient for a reliable measurement of said parameter. Accordingly, a user may readjust the positioning of the apparatus on the skin, such that correct measurement data can be obtained. This general idea is helpful for increasing the measurement security in clinical applications; however, the invention may also be applied to other fields, in which a scattering medium is optically probed by an apparatus as described at the beginning.

According to the invention, such a design offers the advantage that the optical contact detector can detect a detachment of a contact area of the apparatus (normally in skin contact during a measurement) from the skin. This is simply because, in such a situation, the detection light quantity, for example a measured detection light intensity, will be different as compared to a situation in which the contact area is in full skin contact. The latter is the desired situation for optically determining said optical or physiological parameter, which may be a wavelength dependent absorption coefficient of said medium, a concentration of a chromophore in tissue (which is a scattering medium) or a cerebral oxygenation, for example.

In the simplest case, a measured detection light intensity can be taken directly as the variable whose deviation from a target value is computed by the electronic unit. In this case, the target value can be an intensity value and the electronic unit may be configured to issue said alarm signal in case said detection light intensity is measured by said electronic unit to be above said target value.

Generally, the target value and the variable should have the same dimensions and be of the same category to ease the comparison. Hence, the target value may be, for example, a light intensity value or a phase shift value or a time of flight or another parameter such as a concentration of a chromophore or an absorption coefficient. For example, the target value may be a typical detection light intensity measured for the detection wavelength when the contact area of the apparatus is in full skin contact, i.e., in the absence of air gaps.

If the apparatus features several light sources for emitting different measurement wavelengths (for example for enabling multiwavelength NIRS), the contact area may be large. In other words, the contact area may be larger than a sum of individual coupling areas through which the measurement wavelengths are emitted into said medium and/or received from said medium after being backscattered by said medium.

Depending on the variable derived from said detection light quantity, the deviation may be negative or positive.

Using a threshold value, which may be zero in the extreme case or a certain percentage of said target value, for example 5%, offers the advantage of allowing certain variations of said variable without issuing an alarm signal accordingly. For example, even when the contact area is in full skin contact, a measured detection light intensity may vary from patient to patient, due to differences in skin color, liver spots, or tissue composition, to name a few. These variations can be taken into consideration, by issuing the alarm signal only, in case the deviation computed by the electronic unit is greater than said threshold value.

The alarm signal issued by the electronic unit may be a digital or an analogue signal; it can be used, for example, to trigger an optical or acoustical alarm, such that a user of the apparatus becomes aware of the insufficient contact between the apparatus, in particular its contact area, and the skin.

According to the invention, the alarm signal may also be an absence or a drop of a control signal, signifying a good contact (i.e., not worthy of improvement) between the apparatus and the scattering medium, for example tissue.

The term "wavelength", as used above, may be understood as a center wavelength of a broader spectrum emitted by a corresponding light source, for example an LED. For example, an LED emitting a center wavelength of 680 nm within an emission spectrum from 670 nm to 690 nm may be considered as emitting one measurement wavelength of 680 nm, according to the invention. Preferably, the measurement wavelength is located within a transmission window of said medium, because only in this case, a deep penetration into said medium can be achieved, such that information from deep layers of said medium can be obtained.

In other words, the alarm signal may be monitored by the electronic unit. Moreover, the alarm signal, which is indicative of an insufficient skin contact, may be used by the electronic unit to trigger an appropriate reaction. This reaction, which may comprise repeating the measurement of said parameter, may or may not include a warning to the user. Moreover, other control signals may be considered by the electronic unit in conjunction with the alarm signal for triggering said reaction. For example, the alarm signal may be evaluated along with other parameters by a penalty function, and the electronic unit may trigger the reaction according to the result of the penalty function.

The parameter to be measured with the apparatus may be a physiological parameter, for example the oxygenation of tissue or the concentration of a chromophore in said tissue, or an optical parameter, for example an optical attenuation of a measurement wavelength in the medium.

According to the invention, the electronic unit is configured to compute the at least one parameter based on the measured measurement light quantity such that the parameter can be outputted by the apparatus, preferably on a display. The basic approach may be to measure a voltage proportional to the received light intensity and to normalize this voltage for computing a light attenuation based on known source detector separations (SDS). From these values, material parameters such as the absorption coefficient µa (λ) can be computed, for example by solving a system of linear equations.

It is further noted, that the electronic unit may be a single component or consist of multiple components. For example, according to a specific configuration, the apparatus may comprise a mobile electronic device, for example a tablet. This device may be connected by a cable or a wireless connection to a measurement head of the apparatus. In such cases, a first component of the electronic unit may be located inside the measurement head while a second component of the electronic unit may be provided by the mobile electronic device. In other words, part of the calculations necessary for detecting a contact between the apparatus and the skin may be performed by software running on the mobile electronic device. As will be detailed below, the electronic device may also provide a user interface and/or an output device of the apparatus.

An apparatus as introduced above can also be used for applications such as pulsoximetry in which AC- and DC-components are related to each other for determining the pulse frequency. For such applications, the measurement wavelengths may lie in the range from 660 to 940 nm, preferably in the range from 690 nm to 880 nm.

According to the invention, there exist further advantageous embodiments solving the aforementioned problem, which are described in the sub-claims and in the following.

For example, the invention is particularly useful for NIR-oximeters and hence said at least one measurement wavelength lies in the NIR-range from 640 to 1350 nm where light has its maximum depth of penetration in tissue. Penetration depth is to be understood here as the reciprocal of the wavelength dependent absorption factor µₐ(λ), which is an optical parameter characterizing the medium optically probed with the apparatus. In specific applications, which require very high penetration depths, it may be of advantage, if the at least one measurement wavelength lies within a range from 640 nm to 960 nm.

In all these cases, it is of great advantage if the detection wavelength lies outside of the above noted range of 640 to 1350 nm. The reason is simply that a high light attenuation, which is normally to be avoided for the measurement wavelength to achieve high penetration depth, is of advantage for detecting the skin contact of the apparatus with the detection wavelength. The underlying reasoning is that the change in the measured detection light quantity resulting from a varying air gap between the apparatus and the skin will be larger, the higher the detection wavelength is attenuated per unit distance of propagation within the tissue.

It is noted, that the light source and detector may also be implemented by respective light guides. These light guides may guide the detected light to a proximal end of the apparatus, at which electronic devices are located for measuring the intensity of that light.

The term "detector" in the sense of the invention is thus not restricted to electronic devices alone, such as a photodiode or a photomultiplier, but further comprises arrangements of a light detecting surface, such as an end facet of an optical fiber, and corresponding electronic devices for measuring the intensity of said light, for example located at another end facet of said optical fiber. In other words, the interface between the electronic components of the apparatus, which may be solely located in at a proximal end of the apparatus, and the tissue to be optically probed may be based on / delivered by optical fibers. In such a case, no electronic components will be present in a sensor head of the apparatus, but light detecting surfaces of the optical fibers, which serve as "detectors" in the sense of the invention discussed herein.

Likewise, the term "light source" in the sense of the invention comprises not only light emitting devices such as LEDs but also arrangements of a light emitting surface, such as an end facet of an optical fiber, and corresponding light emitting devices, for example located at another end facet of said optical fiber. Thus, a corresponding light emitting device does not necessarily need to be located at the position of a "light source".

For emitting said detection wavelength, the apparatus may feature a detection light source, although this light source is not essential to employing the optical contact detector according to the invention, as will be explained later.

In this case, depending on the application, in particular the depth range probed by the apparatus using the measurement wavelength, said detection light source and the optical contact detector may offer a source-detector-separation of less than 40 mm, preferably of less than 20 mm. In applications in which the penetration depth of the detection wavelength is ultra-short in said medium, this source-detector-separation may be even less than 15 mm or even less than 5 mm. By such dimensioning, it is safeguarded that the optical contact detector can detect the detection wavelength at a reasonable signal-to-noise ratio (SNR).

Alternatively, said detection wavelength may result simply from ambient light. When using ambient light as the detection wavelength, the electronic unit may be configured to detect the detection wavelength using the optical contact detector without using the at least one light source for emitting the at least on measurement wavelength. In other words, prior to the determination of the parameter in the tissue using said light sources, the contact state of the apparatus may be determined exclusively based on ambient light, with all light sources of the apparatus employed in an power-off mode. In such a case, the optical contact detector may selectively detect (by using the optical filter) a detection wavelength within the ambient light that is highly absorbed in the medium. In case, this wavelength from the ambient light can no longer be detected by the apparatus, this may be interpreted by the apparatus as resulting from a good contact state, e.g., a close fit of the apparatus to skin.

The optical contact detector is arranged separate from said at least one detector for detecting said at least one measurement wavelength. In this case, it is preferable if the optical contact detector is located outside of a circle centered around said at least one light source and with a radius that is equal to a distance of said at least one light source from said at least one detector. Likewise, the optical contact detector may be located near an outer edge of said contact area. Such an arrangement offers the advantage that the optical contact detector may detect the ambient light, while the at least one detector may be arranged such that the ambient light does not disturb the measurement of the measurement light quantity, which may be, in particular, a measurement light intensity.

As explained at the beginning, the optical contact detector features an optical filter transmitting the detection wavelength and blocking said at least one measurement wavelength. Due to this filter, the detection of the skin contact can be performed independently of the measurement of the measurement light quantity, i.e., in particular simultaneously. Without such a filter, the detection of the skin contact may be affected in case NIR light propagating through the tissue reaches the optical contact detector, which can lead to a misinterpretation of the contact between the apparatus and the skin at the location of the optical contact detector.

According to a preferred embodiment, the optical filter may therefore be configured to block the complete NIR-range from 640 to 1350 nm, in particular from 600 nm to 1000 nm, most preferably from 600 nm to 1350 nm.

In case the detection wavelength results from an ambient light source, it is of advantage if the filter is configured to block wavelengths of the ambient light source for which the medium to be probed with the apparatus is transparent. This increases the robustness of the contact detection.

On the other hand, optical filters are normally not required on the detector measuring the measurement light quantity, since the intensity resulting from ambient light can be measured first and this measurement can be taken into account when performing the actual measurement of said optical parameter.

The threshold value, which is used by the electronic unit for evaluating the deviation of the measured detection light quantity from the target value, and/or said target value can be pre-defined and/or fix. This is suitable for example, if a static chromophore such as Melanin, whose concentration in tissue is normally static over time, is to be measured as a physiological parameter.

On the other hand, the threshold value and/or the target value can be variable, respectively. A typical application scenario for this case is the measurement of time-varying chromophore, such as oxygenated hemoglobin, whose concentration in the tissue may vary over time.

Furthermore, the threshold value and/or the target value may be adjustable by a user of the apparatus to allow fine-tuning of the contact sensing functionality to a specific application.

As will be explained in more detail below, most preferably, the electronic unit may be configured to adjust the threshold value and/or the target value based on the measurement light quantity and/or based on said parameter, which is computed from the measurement light quantity. This parameter may be, for example, a concentration of a chromophore such as hemoglobin in said medium, as measured by the electronic unit.

When measuring a variable chromophore such as oxygenated / deoxygenated hemoglobin it may be necessary to adjust the threshold value and/or the target value during a measurement. This adjustment can be based on computed concentrations of the chromophore of interest whose concentration is measured with the apparatus.

For example, a green wavelength may be used as the detection wavelength. Based on the concentration of a chromophore measured with NIR-wavelengths, the electronic unit may then compute an expected value for the detection light quantity, in particular for a detection light intensity, and/or the variable derived from the detection light quantity. In this case, it may be advantageous to use a model for the wavelength dependent scattering in the probed medium to calculate the expected value. The expected value for the detection light quantity can then be used by the electronic unit for adjusting the threshold value and/or the target value.

Moreover, different measurement situations may require different threshold values: For example, the attenuation of the detection wavelength may differ, depending on which tissue is to be measured. This is because the tissue on extremities may differ from that on the head of a patient; likewise, the amount of fat in the tissue may change, which may also affect the absorption of the detection wavelength.

A user may therefore fine-tune the threshold value and/or the target value for a specific application. This may be done, for example by offering specific application scenarios that a user may input over a user-interface. The electronic unit may be configured in this case such that, in reaction to an input from a user, the electronic unit may adjust the threshold value and/or the target value accordingly, in particular based on a set of values stored in a storage medium of the apparatus. Simply set, the electronic unit may hence be configured to adjust said threshold value and/or said target value based on a user input.

Furthermore, the threshold value and/or the target value may be fine-tuned by the apparatus. For example, based on a measurement relying on at least two different measurement wavelengths, it is possible to determine a concentration of Hemoglobin in the tissue for computing a tissue oxygen saturation value (StO2). Based on known absorption spectra in the tissue, a nominal value for the light attenuation resulting from the absorption of the detection wavelength by the measured hemoglobin concentration can then be computed and this value can be used to cross-check measurement values obtained for the detection light quantity resulting from the detection wavelength. Here again, it is advantageous to take a model for scattering in the medium into account. This approach allows fine-tuning of the contact-detection-scheme and to issue the alarm signal only, if the skin contact is actually lost.

Simply set, the electronic unit may thus be configured to adjust said threshold value and/or said target value based on a concentration of a chromophore measured with the apparatus. In particular, the apparatus may identify the nature of the probed tissue from its characteristic light attenuation spectrum or from specific characteristics of the measured chromophore composition. The electronic unit may thus be configured to take a characteristic light attenuation spectrum of the medium or a chromophore composition in the medium into account, for adjusting said threshold value and/or said target value.

According to one specific embodiment, the electronic unit may be configured to optically determine specific types of tissue, for example based on a measured ratio of at least two different chromophore concentrations in the tissue (e.g., a ratio of fat to water in the tissue), and to adjust said threshold value and/or said target value according to a determined type of tissue. For this purpose, the electronic unit may access a storage medium of the apparatus on which optimal values for the threshold value and/or target value for various tissue types are stored.

According to another preferred embodiment, the apparatus may feature an output device configured to output, preferably optically and/or acoustically, a warning to a user of said apparatus, in reaction to said issue of said alarm signal. This warning may indicate that a contact between said apparatus, in particular said contact area, and said medium is worthy of improvement.

Preferably, the output device can be an internal or external display, for example the screen of a tablet linked via a cable to the apparatus, visualizing the parameter measured with the apparatus. In a particular embodiment, this display may be configured to change and/or interrupt the rendering of said parameter in reaction to said issue of said alarm signal. Hence, the change of the rendering may indicate a problem with the skin contact of the apparatus.

The electronic unit may be configured to stop and/or repeat a measurement of said parameter in reaction to said issue of said alarm signal.

Furthermore, the electronic unit may be configured to continuously measure said detection light quantity and to initiate a measurement of said parameter only if no alarm signal has been issued.

Yet according to another embodiment, the electronic unit may be configured to continuously measure and output said parameter and to change and/or interrupt said output in reaction to said issue of said alarm signal.

All of these three approaches, which may be used in combination, increase the security and reliability of the optical measurement of the parameter with the apparatus.

As was sketched at the beginning, the target value may be a baseline value measured for said detection light quantity, in particular for a detection light intensity, when said apparatus, in particular said contact area, is in full contact with said medium. Additionally or alternatively, the threshold value may be a certain percentage, e.g. 50%, of said target value.

In addition, as was also discussed before, said medium may be human tissue, in particular including layers of scalp, skull, and cerebral tissue. In this case, it is of particular advantage for a valuable measurement if said at least one measurement wavelength lies within a transmission window of said tissue. And even more of advantage, said detection wavelength may lie within an absorption band of said tissue.

The underlying reason is that a long penetration depth in the tissue is of advantage for measuring said parameter, whereas a short penetration depth is of advantage for an optical contact detection, as was previously explained. In particular, the detection wavelength may be chosen such that a normalized light attenuation of the detection wavelength in said medium is at least a factor of 2, preferably a factor of 5, larger than a corresponding normalized light attenuation of the measurement wavelength in said medium. In this case, the optical contact sensor will be highly sensitive to even small changes of the contact between apparatus and skin.

Since it is favorable to place the detection wavelength in an absorption band of said medium, it may be necessary to employ very short source-detector-separations (SDS), in particular below 5 mm, because otherwise, the measurement signal resulting from said detection wavelength will be drowned in a sea of noise such that no adequate evaluation of that signal is possible. Hence, the separation between said detection light source and said optical contact detector may be less than 5 mm.

For applications such as NIR-oximetry, the detection wavelength can be shorter than 640 nm. In particular, the detection wavelength may belong to the UV-VIS-range from 100 nm to 640 nm.

Alternatively, the detection wavelength can also be longer than 1350 nm. In this case, it is preferable, if the detection wavelength is shorter than 1850 nm to allow efficient contact detection based on absorption of the detection wavelength in the tissue.

The electronic unit may further feature a computing unit configured to compute and output at least one measurement value of said parameter, in particular of a concentration of a chromophore such as HbO, based on measured light intensities resulting from an emission of at least two different measurement wavelengths by said at least one light source. In such a measurement scheme based on at least two different measurement wavelengths, it is to be preferred when the at least two measurement wavelengths are each separated by more than 20 nm and/or lie in the range of 640-960 nm.

According to a preferred embodiment, the apparatus may offer at least four different measurement wavelengths in a measurement range from 640 nm to 880 nm.

As has been outlined before, the electronic unit may be configured to compute an optical or physiological parameter based on said detection light quantity. In this case, said at least one light source and said at least one detector may offer a source-detector-separation of more than 15 mm, preferentially more than 20 mm.

According to another embodiment, the apparatus may feature multiple optical contact sensors detectors each configured to detect a detection light quantity resulting from a detection wavelength, which may be the detection wavelength previously explained or another detection wavelength.

In other words, it is also possible to employ at least two different detection wavelengths for detecting the skin contact of the apparatus. Hence, said issue of the alarm signal may be based on the detection of two different detection wavelengths.

The optical contact detectors may be arranged separately from each other on respective contact regions within said contact area. In this case, the electronic unit may be configured to issue an alarm signal in case any of said contact regions shows a low optical coupling efficiency, based on a measured corresponding detection light quantity.

In accordance with the present invention, there is also provided a method, which solves the afore-mentioned problem. In detail, a method is provided as introduced at the beginning and which is further characterized in that said contact area is brought into contact with said surface, a detection wavelength, which is different from said at least one measurement wavelength and which lies outside of the NIR-range from 640 to 1350 nm, is received through said contact area by said apparatus, and a corresponding detection light quantity resulting from said detection wavelength is measured by said apparatus for evaluating said optical coupling efficiency and to detect the direct skin contact of the apparatus. The measurement wavelength emitted by the apparatus lies within the NIR-range from 640 to 1350 nm and the optical skin contact is detected by employing an optical contact detector which features an optical filter transmitting said detection wavelength and blocking said at least one measurement wavelength; accordingly, using this method, a detection of a skin contact can be performed, independently of, in particular simultaneously to, the measurement of the measurement light quantity. A time-multiplexing approach is thus not necessary.

Preferably, in case a variable derived from said detection light quantity differs from a target value by more than a threshold value, said apparatus may issue an alarm, thereby indicating a low coupling efficiency to a user of the apparatus.

This method delivers the same increased measurement security and advantages previously outlined with respect to the apparatus according to the invention. Ideally, the apparatus, on which this method is applied, may be in particular according to one of the claims directed towards an apparatus and/or as previously explained.

As explained previously, the detection wavelength is received by the optical contact detector after having traveled some distance through the medium, e.g., by scattering. In other words, the detection wavelength may be received through the contact area in a non-reflective measurement mode, in which the detection wavelength reaches the detector (when brought in close contact to said surface) only after having traveled through said medium / tissue for some distance, for example for at least 5 mm, preferably for at least 12 mm. This approach safeguards that a sufficiently high absorption of the detection wavelength in the tissue is realized in the close contact state.

Moreover, it is preferable for robust optical contact detection if the method is performed such that when receiving the measurement wavelength, the light source used for emitting the at least one measurement wavelength is in an power-off mode, i.e. the measurement wavelength is not emitted during the optical contact measurement.

The alarm may be issued in the form of an optical and/or acoustical warning, as has been previously described.

Moreover, the detection wavelength used for evaluating the optical coupling efficiency is different from said at least one measurement wavelength.

According to the invention, the above method can be further optimized:
For example, it is of great advantage to apply the method to NIR-oximetry in which case said measurement wavelength lies within an NIR-range from 640 to 1350 nm and said detection wavelength lies outside of this NIR-range.

As the measurement wavelength, also the detection wavelength may be emitted by said apparatus through said contact area into said medium. This has the advantage, that the contact detector actually measures the contact between the contact area and the skin.

As was previously explained, depending on the desired application, the threshold value and/or said target value may be pre-defined or variable; in particular these values may be respectively adjusted by a user or by said apparatus, preferably by the electronic unit.

Furthermore, in reaction to said issue of said alarm signal,
- a warning may be output, preferably acoustically and/or optically, to a user of the apparatus, in particular by changing and/or interrupting a rendering of said parameter on a display, and/or
- a running measurement of said parameter may be stopped or repeated and/or
- a measurement of said parameter may only be initiated if no alarm signal has been issued, in particular only if said detection light quantity varies from said target value by less than said threshold value, and/or
- a measured value for said parameter may be discarded.

Methods as described above may also be applied to multiple and separate contact regions between said apparatus and said medium, wherein each of said contact regions is illuminated by at least one measurement wavelength emitted by said apparatus and by at least one detection wavelength. This detection wavelength may be likewise emitted by said apparatus or be received from ambient light.

For example, each of these contact regions can be associated with a corresponding local measurement region in said medium. In other words: for each contact region, the apparatus may issue a corresponding alarm signal, in case the corresponding optical coupling efficiency is determined to be low, i.e., worthy of improvement.

Finally, the invention suggests that said detection wavelength can also be employed as a measurement wavelength for measuring said parameter in said medium. In other words, the calculation of said parameter may depend on the measured detection light quantity.

Preferred embodiments of the present invention shall now be described in more detail, although the present invention is not limited to these embodiments: for those skilled in the art it is obvious that further embodiments of the present invention may be obtained by combining features of one or more of the patent claims with each other and/or with one or more features of an embodiment described or illustrated herein. With reference to the accompanying drawings, where features with corresponding technical function are referenced with same numerals even when these features differ in shape or design:
- Fig. 1: shows an apparatus,
- Fig. 2: shows a detailed view on a contact region on the lower side of a sensor head of the apparatus of figure 1,
- Fig. 3: shows a cross-sectional schematic view of the sensor head of figure 2 in touch with a skin surface,
- Fig. 4: shows the cross-sectional schematic view of figure 3, after the contact between sensor head and skin has been improved, and
- Fig. 5: illustrates a schematic top view on the contact region shown in figure 2.

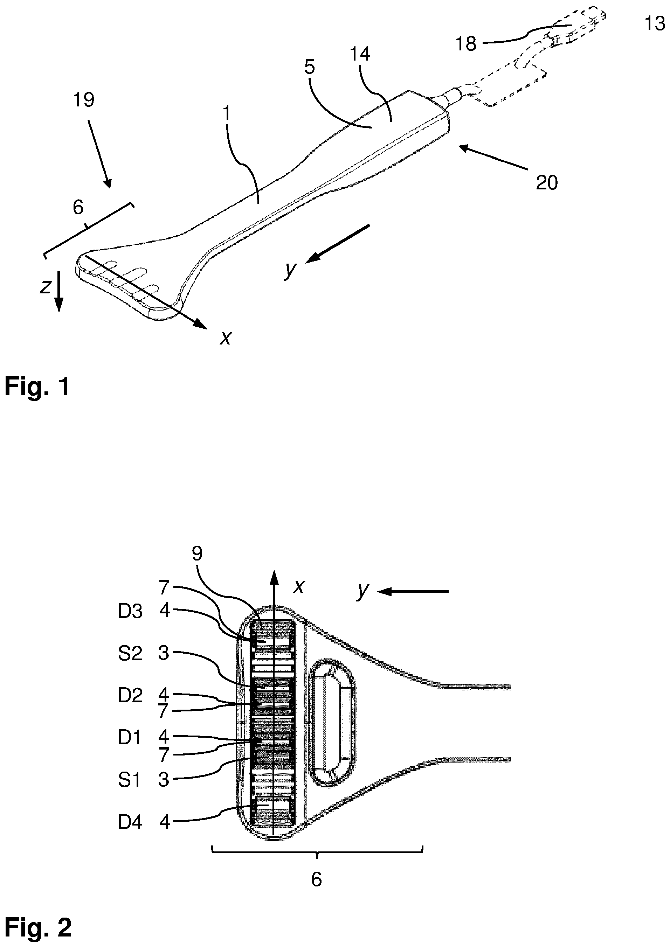

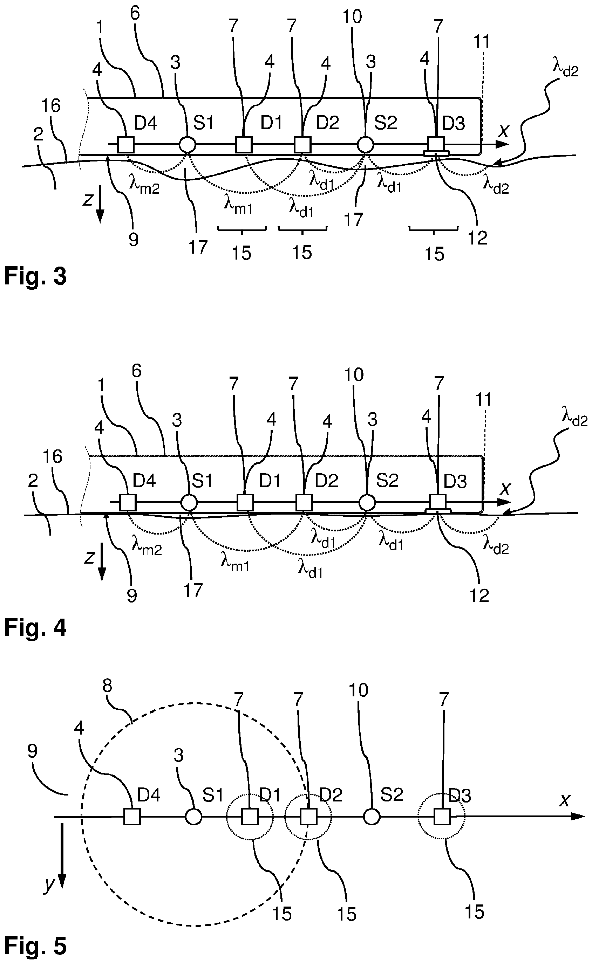

Figure 1 shows an apparatus 1, which can be designed according to the invention, namely an oximeter designed for measuring oxygenation in cerebral tissue for vital-parameter-monitoring of neonates. The apparatus 1 features a bendable sensor head 6 at a distal end 19, bearing several light sources 3 in the form of a linear array of LEDs each emitting a measurement wavelength Ami, respectively, and detectors 4 in the form of single photodiodes. As visible in figure 2, the light sources 3 and detectors 4 are arranged on a contact area 9 on the lower side of the sensor head 6. During a measurement, the contact surface 9 is brought into skin contact on the head of a neonate, as illustrated in figure 3.

As is visible in figure 3, the light source S1 is configured to emit two different measurement wavelengths λm1 and λm2, based on two different LEDs, and these wavelengths are detected by detectors D2 and D4, respectively, after λm1 and λm2 have propagated through the scattering medium 2, which is human tissue in this case, including layers of scalp, skull, and cerebral tissue.

The banana shaped curves, which illustrate an average light path that photons of the respective measurement wavelengths travel through the tissue 2, show that the light is actually backscattered by the medium 2.

The apparatus 1 further features an electronic unit 5, comprising a first component located at a proximal end 20 (c.f. figure 1). A second component of the electronic unit 5 is provided by a tablet as part of the apparatus 1, which is connected to the sensor head 6 of the apparatus 1 shown in figure 1 by a cable 18 (the tablet is not shown in figure 1).

The electronic unit 5 is configured to measure light intensities resulting from the emission of λm1 and λm2 by source S1 into the tissue 2, based on signals which the electronic unit 5 reads-out from the detectors D2 and D4, respectively (c.f. figure 3). Based on these measured intensities, the electronic unit 5 computes a measurement value for the oxygenation of the tissue 2.

This computation is supported by a computing unit 14 of the electronic unit 5, which is configured to compute and output a concentration of oxygenated Hemoglobin based on measured light intensities resulting from an emission of the two measurement wavelengths λm1 and Xm2 by S1 and detected by D2 and D4, respectively.

As is visible in figure 3, the physical contact between the contact area 9 on the lower side of the sensor head 6, through which λm1 and λm2 and λd1 are emitted into the tissue 2 and also received from the tissue 2, and the skin surface 16 of the tissue 2 is quite bad; large air gaps 17 have formed below the light sources S1 and S2, such that only part of the banana-shaped light paths of λm1 and λm2 are actually inside of the tissue 2. As a result, the measured oxygenation does not represent the true value of oxygenation inside the tissue 2.

At the same time, the light path of λd1 shown in figure 3 is also only partly inside of the tissue 2, as compared to the situation of figure 4, in which an acceptable contact between the apparatus 1 and the skin 16 has been re-established and the air gap 17 below source S2 has vanished. Accordingly, the detection light intensity measured by detector D1 will be much higher in the situation of figure 3 as compared to the situation of figure 4, because the detection wavelength λd1 is less attenuated by absorption inside the tissue 2, due to the air gap 17.

As a result, the deviation of the detection light intensity measured in the situation of figure 3, from the target intensity, which may be the detection light intensity measured by D1 in the situation of figure 4 (i.e., when a full contact to the skin has been achieved) will be positive. In other words, a baseline value, measured for the detection light intensity when the contact area 9 is in full skin contact, is used as the target value, i.e., as the target intensity.

In case, the deviation is larger than the threshold value of 25% of said target intensity, the electronic unit 5 issues an alarm signal. Here, the alarm signal is issued in case the detection light intensity is measured by the electronic unit 5 to be above the target value.

As an alternative, the apparatus 1 may determine an estimated concentration of water or another static chromophore such as collagen or fat in the tissue 2 with the detection wavelength λd1, which lies intentionally within an absorption band of H2O. That is, the electronic unit 5 can derive such a concentration as a variable from said detection light intensity detected by the optical contact detectors D1 or D2.

In such a situation, the target value may be a H2O concentration that is representative of the tissue 2. In case the measured H2O-concentration as derived from the detection light intensity deviates by more than a certain threshold value from said target value, the electronic unit will issue an alarm signal accordingly. The reasoning behind this approach is simply that a large deviation of the measured H2O-concentration from a typical concentration is an indicator for an erroneous measurement due to an insufficient skin contact.

As can be seen in figures 2-5, the apparatus further features a third optical contact detector 7, namely detector D3 located near an outer edge 11 of the contact area 9 and separate from the detectors D2 and D4, which are configured to detect the measurement wavelengths λm1 and λm2. D3 is configured to detect a second detection wavelength λd2, which is not emitted by the apparatus 1 but results from ambient light illuminating the tissue 2 near the edge 11, as illustrated in figure 3 and 4. λd2 lies in the visible spectrum, namely at 550 nm, i.e., below 640 nm and within the UV-VIS-range, and is thus different from the first detection wavelength λd1 = 980 nm emitted by the source S2.

As is visible by a comparison of figures 3 and 4, the optical path length traveled by λd2 through the tissue 2 varies, depending on how close the contact between the contact area 9 of the apparatus 1 and the skin 16 is. Hence, D3 can detect an insufficient skin contact, as the detection light intensity resulting from λd2 will be lower in the situation of figure 4, as compared to the situation of figure 3.

D3 features an optical filter 12 which transmits (transmittance > 90%) λd2 = 550 nm and blocks (transmittance of less than 10%) λm1 and λm2. Hence, D3 is not disturbed by light at the two measurement wavelengths λm1 and λm2 propagating in the tissue and reaching D3 and can therefore selectively measure λd2.

The filter 12 may be further designed such that it also transmits λd1. In such a configuration, D3 may also measure λd1 as indicated in figure 3.

Moreover, as is visible in figure 5, the optical contact detector D3 is located outside of a circle 8 centered around light source S1, which emits the measurement wavelengths λm1, and with a radius that is equal to a distance of S1 from detector D2, which detects λm1. Hence, the likeliness that λm1 may disturb the measurement of λd2 by D3 is greatly reduced.

In case the user wants to measure with the oximeter on the skin of an adult, he can input this scenario to the apparatus 1 through a user interface, namely a graphical user interface (GUI) offered by a tablet connected to the sensor head 6 of the apparatus 1 by a cable 18 at the proximal end 20 (c.f. figure 1 - the tablet is not shown). In reaction to this input, the electronic unit 5 automatically adapts the threshold value and the target value. By this approach, the different tissue composition of a neonate and an adult can be taken into account.

The alarm signal issued by the electronic unit 5 triggers a change in the rendering of the oxygenation value measured by detectors D2 and D4 and displayed on the tablet, which serves as an output device 13 of the apparatus 1. By changing the rendering, the apparatus 1 thus outputs a warning to a user in reaction to said issue, thereby indicating to the user that the contact between the contact area 9 and the skin 16 of the tissue 2 is worthy of improvement. Accordingly, the user may therefore readjust the positioning of the apparatus 1 on the skin 16, such that the desired situation of figure 4 is achieved. At the same time, the user becomes aware that the displayed oxygenation value, whose rendering has changed, may be wrong.

Moreover, the issue of the alarm signal triggers the electronic unit 5 to repeat a measurement of the oxygenation in the tissue 2 and to update the displayed oxygenation value accordingly.

As can be seen from the banana shaped curves in figure 3, the apparatus 1 features a detection light source 10, which emits said detection wavelength λd1.

In fact, the apparatus 1 features a second optical contact detector 7, namely detector D2, whose output signals are read-out by the electronic unit 5 for measuring a second detection light intensity, namely the one resulting from the light path of λd1 from S2 to D2. This light path is much shorter than the light path between S2 and the optical contact detector D1, because the source-detector-separation between S2 and D2 is only 5 mm. Hence, the detection light intensity measured by D2 may be used when measuring on tissue which is highly absorbing λd1, a situation in which the detection light intensity measured by D1 may show an insufficient signal-to-noise ratio (SNR), due to the longer light path and hence higher attenuation.

Another advantage of using multiple optical contact detectors 7 is that they may be arranged separately from each other on different contact regions 15 within said contact area 9 described above, as illustrated by the schematic top view on the contact area 9 of figure 5.

The electronic unit 5 of the apparatus 1 is consequently configured to issue an alarm signal in case any of said contact regions 15 shows a low optical coupling efficiency, based on a measured corresponding detection light intensity detected by D1, D2, or D3. Moreover, due to the presence of detectors D3, detecting λd2, as well as D1 and D2, detecting λd1, respectively, the issue of the alarm signal can be based on the detection of the two different detection wavelengths, namely λd2 and λd1. At the same time, due to the absence of an optical filter, detectors D1 and D2 may also be used for detecting the measurement wavelengths λm1 and λm2 emitted by source S1 (not illustrated in figures 3 and 4).

Although not illustrated in figure 5, each of said contact regions 15 may be illuminated by at least one measurement wavelength emitted by the apparatus 1 and by at least one detection wavelength, which may also be emitted by the apparatus 1. This approach ensures that multiple contact regions 15, which are employed in a measurement of said parameter, can be optically monitored by the optical contact detectors 7 with respect to insufficient surface contact, i.e., an insufficient optical coupling efficiency. This further improves the reliability and safety of the optical measurement.

In summary, for improving the safety of optical measurements performed with an apparatus 1 comprising a set of light sources 3 and detectors 4 controlled by an electronic unit 5 for measuring an optical or physiological parameter in a scattering medium 2, in particular human tissue 2, based on a set of measurement wavelengths, it is suggested that the apparatus 1 features at least one optical contact detector 7, for example in the form of a photodiode or a light sensitive end facet of an optical fiber or the like, for measuring a detection light quantity resulting from a detection wavelength, and wherein the apparatus 1 issues an alarm signal, in case the measured light quantity indicates a low optical coupling efficiency between the apparatus 1 and the medium 2.

### List of reference numerals

- 1: apparatus
- 2: scattering medium (e.g. tissue)
- 3: light source
- 4: detector
- 5: electronic unit
- 6: sensor head
- 7: optical contact detector
- 8: circle
- 9: contact area (of 1)
- 10: detection light source
- 11: outer edge (of 9)
- 12: optical filter
- 13: output device
- 14: computing unit
- 15: contact region (within 9)
- 16: surface, e.g. skin (of 2)
- 17: air gap
- 18: cable
- 19: distal end
- 20: proximal end

## Claims

1. **Apparatus (1)** for measuring at least one optical or physiological parameter in human tissue, comprising
- at least one light source (3) configured to emit at least one measurement wavelength within the NIR-range from 640 to 1350 nm,
- at least one detector (4) configured to detect said at least one measurement wavelength, and
- an electronic unit (5) configured
- to measure a measurement light quantity resulting from an emission of said at least one measurement wavelength into said tissue, based on electrical signals read-out from said at least one detector (4), for computing said at least one parameter, and
- to compute said at least one optical or physiological parameter from said measurement light quantity,
- wherein said apparatus (1) features an optical contact detector (7) configured to detect a detection wavelength, which lies outside of the NIR-range from 640 to 1350 nm and is thus different from said at least one measurement wavelength,
- wherein the at least one light source (3), the at least one detector (4) and the optical contact detector (7) are arranged on a contact area (9) of the apparatus (1), in particular on a lower side of a bendable sensor head (6) of the apparatus (1), and
- wherein said contact area (9) is configured to be brought into contact with a skin during a measurement,
- wherein said electronic unit (5) is configured to measure a detection light quantity based on electrical signals outputted by said optical contact detector (7) and to detect a direct skin contact of the contact area (9) based on the measured detection light quantity,
**characterized in that**
- said optical contact detector (7) features an optical filter (12) configured to transmit said detection wavelength and to block said at least one measurement wavelength.

2. Apparatus (1) according to claim 1, wherein said electronic unit (5) is configured to derive a variable from said detection light quantity and to compute a deviation of said variable from a target value,
- preferably and to issue an alarm signal in case said deviation is greater than a threshold value.

3. Apparatus (1) according to one of the preceding claims,
- wherein said detector (4) for detecting said at least one measurement wavelength is positioned at a distance of at least 12 mm, preferably of at least 15 mm, from said at least one light source (3) for emitting at least one measurement wavelength or from a border of a contact area (9) of said apparatus (1).

4. Apparatus according to one of the preceding claims,
- wherein said apparatus (1) features a detection light source (10) for emitting said detection wavelength, in particular wherein said detection light source (10) and said optical contact detector (7) offer a source-detector-separation of less than 40 mm, preferably of less than 20 mm.

5. Apparatus (1) according to claim 2,
- wherein said variable is said detection light quantity,
- preferably wherein said target value is an intensity value and said electronic unit (5) is configured to issue said alarm signal in case said detection light intensity is measured by said electronic unit (5) to be above said target value or
- wherein said electronic unit (5) is configured to compute said variable, which may be an optical absorption or a concentration of a chromophore for example, from said detection light quantity, and/or
- wherein said target value is a light intensity value or a phase shift value or a time of flight.

6. Apparatus (1) according to one of the preceding claims,
- wherein said optical contact detector (7) is separate from said at least one detector (4) for detecting said at least one measurement wavelength.

7. Apparatus (1) according to claim 2,
- wherein said threshold value and/or said target value is/are pre-defined or
- wherein said threshold value and/or said target value is/are variable, in particular adjustable by a user of the apparatus (1) or by the apparatus (1), and/or
- preferably wherein said electronic unit (5) is configured to adjust said threshold value and/or said target value based on said measurement light quantity and/or said parameter or based on a user input.

8. Apparatus (1) according to claim 2,
- wherein said apparatus (1) features an output device (13) configured to output, preferably optically and/or acoustically, a warning to a user of said apparatus (1) in reaction to said issue of said alarm signal,
- preferably wherein said output device (13) is a display visualizing said parameter.

9. Apparatus (1) according to claim 2,
- wherein said electronic unit (5) is configured to stop and/or repeat a measurement of said parameter in reaction to said issue of said alarm signal and/or
- to continuously measure said detection light quantity and to initiate a measurement of said parameter only if no alarm signal has been issued, and/or
- to continuously measure and output said parameter and to change and/or interrupt said output in reaction to said issue of said alarm signal.

10. Apparatus (1) according to claim 2,
- wherein said target value is a baseline value measured for said detection light quantity when said apparatus (1), in particular said contact area (9), is in full contact with the skin and/or
- wherein said threshold value is a certain percentage of said target value.

11. Apparatus (1) according to one of the preceding claims,
- wherein said electronic unit (5) features a computing unit (14) configured to compute and output at least one measurement value of said parameter, in particular a concentration of a chromophore such as HbO, based on measured light intensities resulting from an emission of at least two different measurement wavelengths by said at least one light source (3),
- preferably wherein said at least two different measurement wavelengths are each separated by more than 20 nm and/or lie in the range of 640 nm to 960 nm.

12. Apparatus (1) according to one of the preceding claims,
- wherein said apparatus (1) features multiple optical contact detectors (7) each configured to detect a detection light quantity resulting from a detection wavelength,
- preferably wherein said optical contact detectors (7) are arranged separately from each other on specific contact regions (15) within a contact area (9) of the apparatus (1) and wherein said electronic unit (5) is configured to issue an alarm signal in case any of said contact regions (15) shows a low optical coupling efficiency, based on a measured corresponding detection light quantity.

13. Apparatus (1) according to claim 2, wherein said issue of the alarm signal is based on the detection of two different detection wavelengths.

14. **Method for evaluating an optical coupling efficiency** between a contact area (9) of an apparatus (1) designed for measuring at least one optical or physiological parameter in human tissue and a surface (16) of said tissue,
- in particular wherein said apparatus (1) is according to one of the preceding claims,
- wherein at least one measurement wavelength within the NIR-range from 640 to 1350 nm is emitted by said apparatus (1) through said contact area (9) into said tissue and
- received through said contact area (9) by said apparatus (1) after having propagated through said tissue, and
- wherein a corresponding measurement light quantity resulting from said emission of said measurement wavelength is measured by an electronic unit (5) of said apparatus (1) and the electronic unit (5) computes said at least one optical or physiological parameter from said measurement light quantity,
- wherein at least one light source (3) configured to emit said at least one measurement wavelength, at least one detector (4) configured to detect said at least one measurement wavelength and an optical contact detector (7) are arranged on the contact area (9), in particular on a lower side of a bendable sensor head (6) of the apparatus (1), wherein
- said contact area (9) is brought into contact with said surface (16),
- a detection wavelength lying outside of the NIR-range from 640 to 1350 nm and thus being different from said at least one measurement wavelength is received through said contact area (9) by said apparatus (1),
- in particular after having travelled some distance through said tissue and/or without emitting said measurement wavelength, and
- a corresponding detection light quantity resulting from said detection wavelength is measured by said apparatus (1) for evaluating said optical coupling efficiency and to detect a direct skin contact of the apparatus (1) using the optical contact detector (7),
**characterized in that**
said optical contact detector (7) features an optical filter (12) configured to transmit said detection wavelength and to block said at least one measurement wavelength.

## Patentansprüche

1. Vorrichtung (1) zur Messung mindestens eines optischen oder physiologischen Parameters in menschlichem Gewebe, umfassend
- mindestens eine Lichtquelle (3), die dazu konfiguriert ist, mindestens eine Messwellenlänge innerhalb des NIR-Bereichs von 640 bis 1350 nm zu emittieren,
- mindestens einen Detektor (4), der dazu konfiguriert ist, die mindestens eine Messwellenlänge zu detektieren, und
- eine Elektronikeinheit (5), die dazu konfiguriert ist,
- eine Messlichtmenge, die sich aus einer Emission der mindestens einen Messwellenlänge in das Gewebe ergibt, auf Grundlage elektrischer Signale, die aus dem mindestens einen Detektor (4) ausgelesen werden, zu messen, um den mindestens einen Parameter zu messen, und
- den mindestens einen optischen oder physiologischen Parameter aus der Messlichtmenge zu messen,
- wobei die Vorrichtung (1) einen optischen Kontaktdetektor (7) aufweist, der dazu konfiguriert ist, eine Detektionswellenlänge zu detektieren, die außerhalb des NIR-Bereichs von 640 bis 1350 nm liegt und somit von der mindestens einen Messwellenlänge verschieden ist,
- wobei die mindestens eine Lichtquelle (3), der mindestens eine Detektor (4) und der optische Kontaktdetektor (7) auf einer Kontaktfläche (9) der Vorrichtung (1) angeordnet sind, insbesondere auf einer unteren Seite eines biegsamen Sensorkopfes (6) der Vorrichtung (1), und
- wobei die Kontaktfläche (9) dazu konfiguriert ist, bei einer Messung mit einer Haut in Kontakt gebracht zu werden,
- wobei die Elektronikeinheit (5) dazu konfiguriert ist, ist, eine Detektionslichtmenge auf Grundlage elektrischer Signale, die vom optischen Kontaktdetektor (7) ausgegeben werden, zu messen und einen direkten Hautkontakt der Kontaktfläche (9) auf Grundlage der gemessenen Detektionslichtmenge zu detektieren,
**dadurch gekennzeichnet, dass**
- der optische Kontaktdetektor (7) einen optischen Filter (12) aufweist, der dazu konfiguriert ist, die Detektionswellenlänge durchzulassen und die mindestens eine Messwellenlänge zu blockieren.

2. Vorrichtung (1) nach Anspruch 1, wobei die Elektronikeinheit (5) dazu konfiguriert ist, eine Variable aus der Detektionslichtmenge abzuleiten und eine Abweichung der Variablen von einem Sollwert zu berechnen,
- und vorzugsweise ein Alarmsignal auszugeben, falls die Abweichung größer als ein Schwellenwert ist.

3. Vorrichtung (1) nach einem der vorhergehenden Ansprüche,
- wobei der Detektor (4) zum Detektieren der mindestens einen Messwellenlänge in einem Abstand von mindestens 12 mm, vorzugsweise mindestens 15 mm, von der mindestens einen Lichtquelle (3) zum Emittieren mindestens einer Messwellenlänge oder von einem Rand einer Kontaktfläche (9) der Vorrichtung (1) positioniert ist.

4. Vorrichtung nach einem der vorhergehenden Ansprüche,
- wobei die Vorrichtung (1) eine Detektionslichtquelle (10) zum Emittieren der Detektionswellenlänge aufweist, wobei insbesondere die Detektionslichtquelle (10) und der optische Kontaktdetektor (7) eine Trennung zwischen Quelle und Detektor von weniger als 40 mm bieten, vorzugweise von weniger als 20 mm.

5. Vorrichtung (1) nach Anspruch 2,
- wobei die Variable die Detektionslichtmenge ist,
- wobei vorzugsweise der Sollwert ein Intensitätswert ist und die Elektronikeinheit (5) dazu konfiguriert ist, das Alarmsignal auszugeben, falls die Detektionslichtintensität durch die Elektronikeinheit (5) als über dem Sollwert liegend gemessen wird, oder
- wobei die Elektronikeinheit (5) dazu konfiguriert ist, die Variable, die beispielsweise eine optische Absorption oder eine Konzentration eines Chromophors sein kann, aus der Detektionslichtmenge zu berechnen, und/oder
- wobei der Sollwert ein Lichtintensitätswert oder ein Phasenverschiebungswert oder eine Lichtlaufzeit ist.

6. Vorrichtung (1) nach einem der vorhergehenden Ansprüche,
- wobei der optische Kontaktdetektor (7) getrennt von dem mindestens einen Detektor (4) zum Detektieren der mindestens einen Messwellenlänge ist.

7. Vorrichtung (1) nach Anspruch 2,
- wobei der Schwellenwert und/oder der Sollwert vordefiniert sind/ist oder
- wobei der Schwellenwert und/oder der Sollwert variabel sind/ist, insbesondere von einem Benutzer der Vorrichtung (1) oder durch die Vorrichtung (1) einstellbar, und/oder
- wobei vorzugsweise die Elektronikeinheit (5) dazu konfiguriert ist, den Schwellenwert und/oder den Sollwert auf Grundlage der Messlichtmenge und/oder des Parameters oder auf Grundlage einer Benutzereingabe einzustellen.

8. Vorrichtung (1) nach Anspruch 2,
- wobei die Vorrichtung (1) eine Ausgabeeinheit (13) aufweist, die dazu konfiguriert ist, als Reaktion auf die Ausgabe des Alarmsignals eine Warnung, vorzugsweise optisch und/oder akustisch, an einen Benutzer der Vorrichtung (1) auszugeben,
- wobei vorzugsweise die Ausgabeeinheit (13) eine Anzeige ist, die den Parameter visualisiert.

9. Vorrichtung (1) nach Anspruch 2,
- wobei die Elektronikeinheit (5) dazu konfiguriert ist, als Reaktion auf die Ausgabe des Alarmsignals eine Messung des Parameters anzuhalten und/oder zu wiederholen und/oder
- die Detektionslichtmenge kontinuierlich zu messen und eine Messung des Parameters nur dann einzuleiten, wenn kein Alarmsignal ausgegeben wurde, und/oder
- den Parameter kontinuierlich zu messen und auszugeben und die Ausgabe als Reaktion auf die Ausgabe des Alarmsignals zu ändern und/oder zu unterbrechen.

10. Vorrichtung (1) nach Anspruch 2,
- wobei der Sollwert ein Ausgangswert ist, der für die Detektionslichtmenge gemessen wird, wenn die Vorrichtung (1), insbesondere die Kontaktfläche (9), sich im Vollkontakt mit der Haut befindet und/oder
- wobei der Schwellenwert ein bestimmter Prozentsatz des Sollwerts ist.

11. Vorrichtung (1) nach einem der vorhergehenden Ansprüche,
- wobei die Elektronikeinheit (5) eine Recheneinheit (14) aufweist, die dazu konfiguriert ist, mindestens einen Messwert des Parameters, insbesondere eine Konzentration eines Chromophors wie etwa HbO, auf Grundlage der gemessenen Lichtintensitäten, die sich aus einer Emission von mindestens zwei verschiedenen Messwellenlängen durch die mindestens eine Lichtquelle (3) ergeben, zu berechnen und auszugeben,
- wobei vorzugsweise die mindestens zwei verschiedenen Messwellenlängen jeweils um mehr als 20 nm getrennt sind und/oder im Bereich von 640 nm bis 960 nm liegen.

12. Vorrichtung (1) nach einem der vorhergehenden Ansprüche,
- wobei die Vorrichtung (1) mehrere optische Kontaktdetektoren (7) aufweist, die jeweils dazu konfiguriert sind, eine Detektionslichtmenge, die sich aus einer Detektionswellenlänge ergibt, zu detektieren,
- wobei vorzugsweise die optischen Kontaktdetektoren (7) getrennt voneinander in verschiedenen Kontaktzonen (15) innerhalb einer Kontaktfläche (9) der Vorrichtung (1) angeordnet sind und wobei die Elektronikeinheit (5) dazu konfiguriert ist, auf Grundlage einer gemessenen Detektionslichtmenge ein Alarmsignal auszugeben, falls eine der Kontaktzonen (15) eine geringe optische Kopplungseffizienz zeigt.

13. Vorrichtung (1) nach Anspruch 2, wobei die Ausgabe des Alarmsignals auf Grundlage der Detektion zweier verschiedener Wellenlängen erfolgt.

14. Verfahren zur Auswertung einer optischen Kopplungseffizienz zwischen einer Kontaktfläche (9) einer Vorrichtung (1), die zum Messen mindestens eines optischen oder physiologischen Parameters in menschlichem Gewebe ausgelegt ist, und einer Oberfläche (16) des Gewebes,
- wobei insbesondere die Vorrichtung (1) nach einem der vorhergehenden Ansprüche beschaffen ist,
- wobei mindestens eine Messwellenlänge innerhalb des NIR-Bereichs von 640 bis 1350 nm von der Vorrichtung (1) durch die Kontaktfläche (9) in das Gewebe emittiert wird und
- nach dem Verbreiten durch das Gewebe durch die Kontaktfläche (9) von die Vorrichtung (1) empfangen wird und
- wobei die entsprechende Messlichtmenge, die sich aus der Emission der Messwellenlänge ergibt, durch eine Elektronikeinheit (5) der Vorrichtung (1) gemessen wird und die Elektronikeinheit (5) den mindestens einen optischen oder physiologischen Parameter aus der Messlichtmenge berechnet,
- wobei die mindestens eine Lichtquelle (3), die dazu konfiguriert ist, die mindestens eine Messwellenlänge zu emittieren, mindestens ein Detektor (4), der dazu konfiguriert ist, die mindestens eine Messwellenlänge zu detektieren, und ein optischer Kontaktdetektor (7) auf der Kontaktfläche (9) angeordnet sind, insbesondere auf einer unteren Seite eines biegsamen Sensorkopfes (6) der Vorrichtung (1), wobei
- die Kontaktfläche (9) in Kontakt mit der Oberfläche (16) gebracht wird,
- eine Detektionswellenlänge, die außerhalb des NIR-Bereichs von 640 bis 1350 nm liegt und somit von der mindestens einen Messwellenlänge verschieden ist, durch die Kontaktfläche (9) von der Vorrichtung (1) empfangen wird,
- insbesondere nachdem sie einen Weg durch das Gewebe zurückgelegt hat und/oder ohne die Messwellenlänge zu emittieren, und
- eine entsprechende Detektionslichtmenge, die sich aus der Detektionswellenlänge ergibt, durch die Vorrichtung (1) gemessen wird, um die optische Kopplungseffizienz zu bewerten und einen direkten Hautkontakt der Vorrichtung (1) mithilfe des optischen Kontaktdetektors (7) zu detektieren,
**dadurch gekennzeichnet, dass**
der optische Kontaktdetektor (7) einen optischen Filter (12) aufweist, der dazu konfiguriert ist, die Detektionswellenlänge durchzulassen und die mindestens eine Messwellenlänge zu blockieren.

## Revendications

1. Appareil (1) pour mesurer au moins un paramètre optique ou physiologique dans un tissu humain, comprenant
- au moins une source de lumière (3) configurée pour émettre au moins une longueur d'onde de mesure dans la plage NIR allant de 640 à 1 350 nm,
- au moins un détecteur (4) configuré pour détecter ladite au moins une longueur d'onde de mesure, et
- une unité électronique (5) configurée
- pour mesurer une quantité de lumière de mesure résultant d'une émission de ladite au moins une longueur d'onde de mesure dans ledit tissu, sur la base de signaux électriques lus à partir dudit au moins un détecteur (4) pour calculer ledit au moins un paramètre, et
- pour calculer ledit au moins un paramètre optique ou physiologique à partir de ladite quantité de lumière de mesure,
- dans lequel ledit appareil (1) présente un détecteur de contact optique (7) configuré pour détecter une longueur d'onde de détection, qui se trouve en dehors de la plage NIR de 640 à 1 350 nm et est donc différente de ladite au moins une longueur d'onde de mesure,
- dans lequel la au moins une source de lumière (3), le au moins un détecteur (4) et le détecteur de contact optique (7) sont agencés sur une zone de contact (9) de l'appareil (1), en particulier sur un côté inférieur d'une tête de capteur pliable (6) de l'appareil (1), et
- dans lequel ladite zone de contact (9) est configurée pour être mise en contact avec une peau lors d'une mesure,
- dans lequel ladite unité électronique (5) est configurée pour mesurer une quantité de lumière de détection sur la base de signaux électriques délivrés en sortie par ledit détecteur de contact optique (7) et pour détecter un contact direct avec la peau de la zone de contact (9) sur la base de la quantité de lumière de détection mesurée, **caractérisé en ce que**
- ledit détecteur de contact optique (7) présente un filtre optique (12) configuré pour transmettre ladite longueur d'onde de détection et pour bloquer ladite au moins une longueur d'onde de mesure.

2. Appareil (1) selon la revendication 1, dans lequel ladite unité électronique (5) est configurée pour déduire une variable à partir de ladite quantité de lumière de détection et pour calculer un écart de ladite variable à partir d'une valeur cible,
- de préférence, et pour délivrer un signal d'alarme dans le cas où ledit écart est supérieur à une valeur de seuil.

3. Appareil (1) selon l'une quelconque des revendications précédentes,
- dans lequel ledit détecteur (4) pour détecter ladite au moins une longueur d'onde de mesure est positionné à une distance d'au moins 12 mm, de préférence d'au moins 15 mm, de ladite au moins une source de lumière (3) pour émettre au moins une longueur d'onde de mesure, ou d'une bordure d'une zone de contact (9) dudit appareil (1).

4. Appareil selon l'une quelconque des revendications précédentes,
- dans lequel ledit appareil (1) présente une source de lumière de détection (10) pour émettre ladite longueur d'onde de détection, en particulier dans lequel ladite source de lumière de détection (10) et ledit détecteur de contact optique (7) offrent une séparation source-détecteur de moins de 40 mm, de préférence de moins de 20 mm.

5. Appareil (1) selon la revendication 2,
- dans lequel ladite variable est ladite quantité de lumière de détection,
- de préférence dans lequel ladite valeur cible est une valeur d'intensité et ladite unité électronique (5) est configurée pour délivrer ledit signal d'alarme au cas où ladite intensité de lumière de détection soit mesurée par ladite unité électronique (5) comme étant supérieure à ladite valeur cible, ou
- dans lequel ladite unité électronique (5) est configurée pour calculer ladite variable, qui peut être une absorption optique ou une concentration d'un chromophore, par exemple, à partir de ladite quantité de lumière de détection, et/ou
- dans lequel ladite valeur cible est une valeur d'intensité lumineuse ou une valeur de déphasage ou un temps de vol.

6. Appareil (1) selon l'une quelconque des revendications précédentes,
- dans lequel ledit détecteur de contact optique (7) est séparé dudit au moins un détecteur (4) pour détecter ladite au moins une longueur d'onde de mesure.

7. Appareil (1) selon la revendication 2,
- dans lequel ladite valeur de seuil et/ou ladite valeur cible est/sont prédéfinies, ou
- dans lequel ladite valeur de seuil et/ou ladite valeur cible est/sont des variables, notamment ajustables par un utilisateur de l'appareil (1) ou par l'appareil (1), et/ou
- de préférence dans lequel ladite unité électronique (5) est configurée pour ajuster ladite valeur de seuil et/ou ladite valeur cible sur la base de ladite quantité de lumière de mesure et/ou dudit paramètre ou sur la base d'une entrée d'utilisateur.

8. Appareil (1) selon la revendication 2,
- dans lequel ledit appareil (1) présente un dispositif de sortie (13) configuré pour délivrer en sortie, de préférence optiquement et/ou acoustiquement, un avertissement à un utilisateur dudit appareil (1) en réaction à ladite délivrance dudit signal d'alarme,
- de préférence, dans lequel ledit dispositif de sortie (13) est un affichage permettant de visualiser ledit paramètre.

9. Appareil (1) selon la revendication 2,
- dans lequel ladite unité électronique (5) est configurée pour arrêter et/ou répéter une mesure dudit paramètre en réaction à ladite délivrance dudit signal d'alarme, et/ou
- pour mesurer en continu ladite quantité de lumière de détection et pour lancer une mesure dudit paramètre uniquement si aucun signal d'alarme n'a été délivré, et/ou
- pour mesurer et délivrer en sortie en continu ledit paramètre et pour modifier et/ou interrompre ladite délivrance en sortie en réaction à ladite délivrance dudit signal d'alarme.

10. Appareil (1) selon la revendication 2,
- dans lequel ladite valeur cible est une valeur de référence mesurée pour ladite quantité de lumière de détection lorsque ledit appareil (1), en particulier ladite zone de contact (9), est en plein contact avec la peau et/ou
- dans lequel ladite valeur de seuil est un certain pourcentage de ladite valeur cible.

11. Appareil (1) selon l'une quelconque des revendications précédentes,
- dans lequel ladite unité électronique (5) présente une unité de calcul (14) configurée pour calculer et délivrer en sortie au moins une valeur de mesure dudit paramètre, en particulier une concentration d'un chromophore tel que HbO, sur la base des intensités de lumière mesurées résultant d'une émission d'au moins deux longueurs d'onde de mesure différentes par ladite au moins une source de lumière (3),
- de préférence dans lequel lesdites au moins deux longueurs d'onde de mesure différentes sont chacune séparées de plus de 20 nm et/ou se situent dans la plage de 640 nm à 960 nm.

12. Appareil (1) selon l'une quelconque des revendications précédentes,
- dans lequel ledit appareil (1) présente de multiples détecteurs de contact optiques (7) configurés chacun pour détecter une quantité de lumière de détection résultant d'une longueur d'onde de détection,
- de préférence dans lequel lesdits détecteurs de contact optiques (7) sont agencés séparément les uns des autres sur des régions de contact spécifiques (15) à l'intérieur d'une zone de contact (9) de l'appareil (1) et dans lequel ladite unité électronique (5) est configurée pour délivrer un signal d'alarme au cas où l'une quelconque desdites régions de contact (15) présente une faible efficacité de couplage optique, sur la base d'une quantité de lumière de détection correspondante mesurée.

13. Appareil (1) selon la revendication 2, dans lequel ladite délivrance du signal d'alarme est basée sur la détection de deux longueurs d'onde de détection différentes.

14. Procédé pour évaluer une efficacité de couplage optique entre une zone de contact (9) d'un appareil (1) conçu pour mesurer au moins un paramètre optique ou physiologique dans un tissu humain et une surface (16) dudit tissu,
- en particulier dans lequel ledit appareil (1) est selon l'une quelconque des revendications précédentes,
- dans lequel au moins une longueur d'onde de mesure dans la plage NIR de 640 à 1 350 nm est émise par ledit appareil (1) à travers ladite zone de contact (9) jusque dans ledit tissu, et
- reçue à travers ladite zone de contact (9) par ledit appareil (1) après s'être propagée à travers ledit tissu, et
- dans lequel une quantité de lumière de mesure correspondante résultant de ladite émission de ladite longueur d'onde de mesure est mesurée par une unité électronique (5) dudit appareil (1) et l'unité électronique (5) calcule ledit au moins un paramètre optique ou physiologique à partir de ladite quantité de lumière de mesure,
- dans lequel au moins une source de lumière (3) configurée pour émettre ladite au moins une longueur d'onde de mesure, au moins un détecteur (4) configuré pour détecter ladite au moins une longueur d'onde de mesure et un détecteur de contact optique (7) sont agencés sur la zone de contact (9), en particulier sur un côté inférieur d'une tête de capteur pliable (6) de l'appareil, dans lequel
- ladite zone de contact (9) est amenée en contact avec ladite surface (16),
- une longueur d'onde de détection située en dehors de la plage NIR de 640 à 1 350 nm et donc différente de ladite au moins une longueur d'onde de mesure est reçue à travers ladite zone de contact (9) par ledit appareil (1),
- en particulier après avoir parcouru une certaine distance à travers ledit tissu et/ou sans émettre ladite longueur d'onde de mesure, et
- une quantité de lumière de détection correspondante résultant de ladite longueur d'onde de détection est mesurée par ledit appareil (1) pour évaluer ladite efficacité de couplage optique et pour détecter un contact direct avec la peau de l'appareil (1) en utilisant le détecteur de contact optique (7), **caractérisé en ce que**
ledit détecteur de contact optique (7) présente un filtre optique (12) configuré pour transmettre ladite longueur d'onde de détection et pour bloquer ladite au moins une longueur d'onde de mesure.
